# EUROPEAN PATENT APPLICATION

(11) **EP 2 735 572 A1**
(43) Date of publication of application: **28.05.2014**
(21) Application number: 13193912.6
(22) Date of filing: 21.11.2013
(51) Int. Cl.: C07K 16/30

(54) **Monoclonal antibody to human epithelial cell adhesion molecule and method for detecting circulating tumor cells using the same**

(30) Priority: 22.11.2012 JP 2012256774
(71) Applicant: Nihon Kohden Corporation, Shinjuku-ku Tokyo 161-8560 (JP)
(72) Inventor: Nagai, Yutaka, Tokyo, 161-8560 (JP); Takao, Masashi, Miyagi, 980-0874 (JP)
(74) Representative: Rogers, Alex Lee

(57) **Abstract**

An anti-EpCAM monoclonal antibody is provided which specifically recognizes an epitope different from the epitope which conventionally known anti-EpCAM monoclonal antibodies recognize (the N-terminal EGF-like domain). Also provided is a hybridoma which is capable of producing such monoclonal antibody, and a method for detecting CTC using such monoclonal antibody and a kit for detecting CTC, which is used for the method. The present application provides a monoclonal antibody to the human epithelial cell adhesion molecule (EpCAM) as a solution for the above problem, wherein the monoclonal antibody to the human epithelial cell adhesion molecule (EpCAM) specifically reacts with the CP region of the human epithelial cell adhesion molecule (EpCAM) and is produced by a hybridoma with accession No. NITE BP-1449. There are also provided a method for detecting circulating tumor cells using the above antibody and a kit for detecting circulating tumor cells, which is used for the method, and a hybridoma with accession No. NITE BP-1449.

SEQUENCE LISTING

<110>  NIHON KOHDEN CORPORATION

<120>  MONOCLONAL ANTIBODY AGAINST HUMAN-DERIVED EPITHELIAL CELL
       ADHESION MOLECULE AND METHOD FOR DETECTING CIRCULATING TUMOR
       CELLS BY USING THE SAME

<130>  F13-202

<160>  5

<170>  PatentIn version 3.5

<210>  1
<211>  945
<212>  DNA
<213>  Homo sapiens

<400>  1
atggcgcccc cgcaggtcct cgcgttcggg cttctgcttg ccgcggcgac ggcgactttt      60

gccgcagctc aggaagaatg tgtctgtgaa aactacaagc tggccgtaaa ctgctttgtg     120

aataataatc gtcaatgcca gtgtacttca gttggtgcac aaaatactgt catttgctca     180

aagctggctg ccaaatgttt ggtgatgaag gcagaaatga atggctcaaa acttgggaga 240

agagcaaaac ctgaaggggc cctccagaac aatgatgggc tttatgatcc tgactgcgat 300

gagagcgggc tctttaaggc caagcagtgc aacggcacct ccatgtgctg gtgtgtgaac 360

actgctgggg tcagaagaac agacaaggac actgaaataa cctgctctga gcgagtgaga 420

acctactgga tcatcattga actaaaacac aaagcaagag aaaaacctta tgatagtaaa 480

agtttgcgga ctgcacttca gaaggagatc acaacgcgtt atcaactgga tccaaaattt 540

atcacgagta ttttgtatga gaataatgtt atcactattg atctggttca aaattcttct 600

caaaaaactc agaatgatgt ggacatagct gatgtggctt attattttga aaaagatgtt 660

aaaggtgaat ccttgtttca ttctaagaaa atggacctga cagtaaatgg ggaacaactg 720

gatctggatc ctggtcaaac tttaatttat tatgttgatg aaaaagcacc tgaattctca 780

atgcagggtc taaaagctgg tgttattgct gttattgtgg ttgtggtgat agcagttgtt 840

gctggaattg ttgtgctggt tatttccaga aagaagagaa tggcaaagta tgagaaggct 900

gagataaagg agatgggtga gatgcatagg gaactcaatg cataa 945

<210> 2

<211> 314

<212> PRT

<213> Homo sapiens

<400> 2

Met Ala Pro Pro Gln Val Leu Ala Phe Gly Leu Leu Leu Ala Ala Ala

1               5                   10                  15

Thr Ala Thr Phe Ala Ala Ala Gln Glu Glu Cys Val Cys Glu Asn Tyr

            20                  25                  30

Lys Leu Ala Val Asn Cys Phe Val Asn Asn Asn Arg Gln Cys Gln Cys

        35                  40                  45

Thr Ser Val Gly Ala Gln Asn Thr Val Ile Cys Ser Lys Leu Ala Ala

    50                  55                  60

Lys Cys Leu Val Met Lys Ala Glu Met Asn Gly Ser Lys Leu Gly Arg

65                  70                  75                  80

Arg Ala Lys Pro Glu Gly Ala Leu Gln Asn Asn Asp Gly Leu Tyr Asp

                85                  90                  95

Pro Asp Cys Asp Glu Ser Gly Leu Phe Lys Ala Lys Gln Cys Asn Gly

            100                 105                 110

Thr Ser Met Cys Trp Cys Val Asn Thr Ala Gly Val Arg Arg Thr Asp

        115                 120                 125

Lys Asp Thr Glu Ile Thr Cys Ser Glu Arg Val Arg Thr Tyr Trp Ile

    130                 135                 140

Ile Ile Glu Leu Lys His Lys Ala Arg Glu Lys Pro Tyr Asp Ser Lys

145                 150                 155                 160

Ser Leu Arg Thr Ala Leu Gln Lys Glu Ile Thr Thr Arg Tyr Gln Leu

                165                 170                 175

Asp Pro Lys Phe Ile Thr Ser Ile Leu Tyr Glu Asn Asn Val Ile Thr

            180                 185                 190

Ile Asp Leu Val Gln Asn Ser Ser Gln Lys Thr Gln Asn Asp Val Asp

        195                 200                 205

Ile Ala Asp Val Ala Tyr Tyr Phe Glu Lys Asp Val Lys Gly Glu Ser

    210                 215                 220

Leu Phe His Ser Lys Lys Met Asp Leu Thr Val Asn Gly Glu Gln Leu

225                 230                 235                 240

Asp Leu Asp Pro Gly Gln Thr Leu Ile Tyr Tyr Val Asp Glu Lys Ala

                245                 250                 255

Pro Glu Phe Ser Met Gln Gly Leu Lys Ala Gly Val Ile Ala Val Ile

            260                 265                 270

Val Val Val Val Ile Ala Val Val Ala Gly Ile Val Val Leu Val Ile

        275                 280                 285

Ser Arg Lys Lys Arg Met Ala Lys Tyr Glu Lys Ala Glu Ile Lys Glu

    290                 295                 300

Met Gly Glu Met His Arg Glu Leu Asn Ala

305                 310

<210> 3

<211> 945

<212> DNA

<213> Artificial Sequence

<220>

<223>  cDNA sequence encoding Hyb. 1

<400>  3

atggcgcccc cgcaggtcct cgcgttcggg cttctgcttg ccgcggcgac ggcgactttt      60

gccgcagctc agagagactg tgtctgtgac aactacaagc tggcaacaag ttgctctctg     120

aatgaatatg gtgaatgcca gtgtacttcc tatggtacac agaatactgt catttgctcc     180

aaactggcgg ccaaatgttt ggtgatgaag gcagaaatga atggctcaaa acttgggaga     240

agagcaaaac ctgaaggggc cctccagaac aatgatgggc tttatgatcc tgactgcgat     300

gagagcgggc tctttaaggc caagcagtgc aacggcacct ccacgtgctg gtgtgtgaac     360

actgctgggg tcagaagaac agacaaggac actgaaataa cctgctctga gcgagtgaga     420

acctactgga tcatcattga actaaaacac aaagcaagag aaaaacctta tgatagtaaa     480

agtttgcgga ctgcacttca gaaggagatc acaacgcgtt atcaactgga tccaaaattt     540

atcacgagta ttttgtatga gaataatgtt atcactattg atctggttca aaattcttct     600

caaaaaactc agaatgatgt ggacatagct gatgtggctt attattttga aaaagatgtt 660

aaaggtgaat ccttgtttca ttctaagaaa atggacctga cagtaaatgg ggaacaactg 720

gatctggatc ctggtcaaac tttaatttat tatgttgatg aaaaagcacc tgaattctca 780

atgcagggtc taaaagctgg tgttattgct gttattgtgg ttgtggtgat agcagttgtt 840

gctggaattg ttgtgctggt tatttccaga aagaagagaa tggcaaagta tgagaaggct 900

gagataaagg agatgggtga gatgcatagg gaactcaatg cataa 945

<210> 4

<211> 945

<212> DNA

<213> Artificial Sequence

<220>

<223> cDNA sequence encoding Hyb. 2

<400> 4

atggcgcccc cgcaggtcct cgcgttcggg cttctgcttg ccgcggcgac ggcgactttt 60

gccgcagctc aggaagaatg tgtctgtgaa aactacaagc tggccgtaaa ctgctttgtg 120

aataataatc gtcaatgcca gtgtacttca gttggtgcac aaaatactgt catttgctca 180

aagcttgcgt ctaaatgctt ggcgatgaaa gcagaaatga ctcacagcaa gtctgggagg 240

aggataaagc ccgaaggggc gatccagaac aacgatgggc tgtacgaccc cgactgcgac 300

gagcaggggc tcttcaaagc caagcagtgc aacggcaccg ccacgtgctg gtgtgtgaac 360

actgctgggg tcagaagaac agacaaggac actgaaataa cctgctctga gcgagtgaga 420

acctactgga tcatcattga actaaaacac aaagcaagag aaaaacctta tgatagtaaa 480

agtttgcgga ctgcacttca gaaggagatc acaacgcgtt atcaactgga cccaaaattt 540

atcacgagta ttttgtatga gaataatgtt atcactattg atctggttca aaattcttct 600

caaaaaactc agaatgatgt ggacatagct gatgtggctt attattttga aaaagatgtt 660

aaaggtgaat ccttgtttca ttctaagaaa atggacctga cagtaaatgg ggaacaactg 720

gatctggacc ctggtcaaac tttaatttat tatgttgatg aaaaagcacc tgaattctca 780

atgcagggtc taaaagctgg tgttattgct gttattgtgg ttgtggtgat agcagttgtt 840

gctggaattg ttgtgctggt tatttccaga aagaagagaa tggcaaagta tgagaaggct 900

gagataaagg agatgggtga gatgcatagg gaactcaatg cataa 945

<210> 5

<211> 948

<212> DNA

<213> Artificial Sequence

<220>

<223> cDNA sequence encoding Hyb. 3

<400> 5

atggcgcccc cgcaggtcct cgcgttcggg cttctgcttg ccgcggcgac ggcgactttt 60

gccgcagctc aggaagaatg tgtctgtgaa aactacaagc tggccgtaaa ctgctttgtg 120

aataataatc gtcaatgcca gtgtacttca gttggtgcac aaaatactgt catttgctca 180

aagctggctg ccaaatgttt ggtgatgaag gcagaaatga atggctcaaa acttgggaga 240

agagcaaaac ctgaaggggc cctccagaac aatgatgggc tttatgatcc tgactgcgat 300

gagagcgggc tctttaaggc caagcagtgc aacggcacct ccacgtgctg gtgtgtcaac 360

accgccggag tccgaagaac cgacaaggac acggagatca cgtgctccga gcgcgtgagg 420

acctactgga tcatcattga actaaaacac aaagaaagag aaagccccta cgaccatcag 480

agcttgcaga ctgcgcttca agaggcgttc acatctcgat ataagctgaa tcagaaattt 540

atcaaaaaca ttatgtatga gaataatgtt atcaccattg atctgatgca aaactcttct 600

cagaaaacac aagacgacgt ggacatagct gatgtggctt actattttga aaaagatgtg 660

aagggggagt ccctgttcca ttcttctaag agcatggacc tgagagtgaa cggagagccg 720

ctcgatctgg accccgggca gactctgatt tactacgttg atgaaaaggc acccgaattc 780

tcaatgcagg gtctaaaagc tggtgttatt gctgttattg tggttgtggt gatagcagtt 840

gttgctggaa ttgttgtgct ggttatttcc agaaagaaga gaatggcaaa gtatgagaag 900

gctgagataa aggagatggg tgagatgcat agggaactca atgcataa 948

## Description

### BACKGROUND

The present invention relates to a monoclonal antibody to the human epithelial cell adhesion molecule and a method for detecting circulating tumor cells using the same.

Circulating Tumor Cells (CTC) are defined as tumor cells which circulate through the peripheral blood flow of cancer patients and are tumor cells which infiltrate from a primary tumor or a metastatic tumor into blood vessels. The CTC detection has attracted attention as one of methods for early detection of metastatic malignant tumors in recent years. The reason why is that the CTC detection can diagnose metastatic malignant tumors less invasively and more accurately than X-ray photographs and the detection of tumor markers in blood serum, and can be used for prediction of patient prognosis and as an index of therapeutic effects.

CTC are very rare cells, and it is known that only about one cell exists in 10⁹ to 10¹⁰ cells/mL of blood cells contained in blood of a patient with metastatic cancer. Because of this, much effort has been focused on technique development to accurately detect rare CTC from peripheral blood. The main detection methods which have been previously developed include an immunohistochemical method, a PCR method, a flow cytometry method and the like. As described above, however, CTC are very rare cells, and it is required that CTC be enriched to a level according to a detection method.

Among various methods which have been developed as a method for enriching CTC, the most widely used method is the enrichment of tumor cells which express epithelial cell-specific antigens on cell surfaces. Many of them use an immunomagnetic enrichment with magnetic beads, on which a monoclonal antibody to an epithelial cell adhesion molecule (EpCAM; also referred to as "CD326") is immobilized. After mixing the antibody-conjugated beads with blood sample, the beads-coated target cells are enriched in a magnetic field.

To obtain efficient, accurate, and reproducible data for the CTC detection/determination, a consistent reagent kit and/or an integrated device will be ideal. Multi-step handling operations, for example operations such as cell staining, washing, separation and dispensing, cause a loss of CTC, and thus it is desired that these operations be avoided as much as possible. In addition, it is also desired a system which can consistently carry out analysis in an integral detection device be used. CellSearch® System (Veridex) is the only FDA- approved device as a CTC detection device and is used to predict progression-free survival rate (PFS) and overall survival rate (OS) of metastatic breast cancer, colon cancer and prostate cancer. In this device, CTC are enriched by magnetic beads on which an anti-EpCAM antibody is immobilized, and tumor cells are immunostained, and the number of tumor cells is then counted using an automatic fluorescence microscope. More specifically, 7.5 mL blood sample is first transferred to a conical tube, and after mingling this with a buffer solution, the erythrocyte layer is separated and removed by a centrifuge. After that, tumor cells from epithelial origin (i.e. carcinoma cells migrated into blood stream) are specifically separated and extracted from many other blood cells by immunomagnetic enrichment fashion, where EpCAM conjugated superparamagnetic beads are incubated with the serum. Next, a cocktail of fluorescently-labeled cytokeratin monoclonal antibodies is allowed to react with the separated epithelial cells, and simultaneously the nuclei of the cells are stained using a fluorescent DNA staining substance (DAPI). At this time, to distinguish contaminated leukocytes from CTC, a fluorescently-labeled anti-CD45 antibody is allowed to react therewith. Thus, CTC in a blood sample is detected and determined.

In the detection and determination method for CTC in a blood sample by the above-described CellSearch® System, the treatment of cell immobilization and cell membrane penetration has been required, since cytokeratins are intracellular components. Therefore, a sample analyzed by CellSearch® System cannot be used for further downstream analyses (e.g., gene diagnosis and genome analysis such as Reverse Transcription-Polymerase Chain Reaction; RT-PCR, chromosome aneuploidy test and mutational analysis).

In order to perform further downstream CTC analyses from the detected and enumerated tumor cells, the whole process of the enrichment and detection/enumeration of CTC is required keeping cellular integrity intact. As a promising solution for the non-destructive CTC test, intact CTC enumeration and analysis procedure (iCeap) has been proposed in, for example, Takao et al., Cytometry, Part A, 79A: 107-117, 2011 (hereinafter, referred to as Non-Patent Document 1).

As with the CellSearch system, iCeap also targets EpCAM expressed on CTC surfaces, but is different from the CellSearch system in terms of avoiding membrane permeabilization followed by immunostaining of the intracellular cytokeratins. In iCeap, one of two different types of anti-EpCAM monoclonal antibody to be used is bound to magnetic beads, and another one is fluorescently-labeled (hereinafter, of two different types of anti-EpCAM monoclonal antibody used in iCeap, the monoclonal antibody which is bound to magnetic beads may be referred to as "a first antibody" and that which is fluorescently-labeled may be referred to as "a second antibody"). The second antibody plays a role of a CTC marker in the detection/enumeration process.

iCeap, is carried out as follows, for example. First, erythrocytes in a blood sample are lyzed, followed by centrifugation, and the supernatant is then removed to obtain a pellet. A first antibody-magnetic beads complex and a fluorescently-labeled second antibody are allowed to come in contact with the pellet. In Non-Patent Document 1, HEA-125 is used as the first antibody and EBA-1 is used as the second antibody. In this case, both the first antibody and the second antibody specifically recognize EpCAM protein expressed on CTC surfaces and are bound thereto. Next, CTC are subjected to magnetic enrichment by MACS magnetic cell separation system. Strategy of the CTC enrichment with the first antibody-magnetic beads and the exclusion of white blood cells by CD45 antibody resemble those of CellSearch® System while strategy of the CTC detection/enumeration is different between CellSearch® System and iCeap. Although the former requires membrane-permeabilization in order to stain the intracellular cytokeratins (CKs) by anti-CK antibodies, the latter can omit this process. Thus, the iCeap method enables intact CTC enumeration. Keeping the intactness, they introduced discrimination of live or dead CTC by using DNA staining dyes with memebrane-peremeant or impermeant nature (i.e. the membrane-permeant dye stains both live and dead cells while the membrane-impermeant dye stains only dead cells). Using the sample thus obtained, analysis is carried out by flow cytometry, thereby being able to detect and determine each living/died CTC in distinction to nonspecific binding to leukocytes.

The detection and determination of CTC by this iCeap hardly affects viability of cells contained in a sample, and thus unlike CellSearch® system, a sample after completion of analysis by iCeap can be used for further downstream analyses (e.g., gene diagnosis and genome analysis such as RT-PCR, a chromosome aneuploidy test and mutational analysis). In at least this respect, iCeap can be said to be an excellent analysis technique having an advantage which the CellSearch system does not have.

As described above, HEA-125 antibody is used as the first antibody and EBA-1 antibody is used as the second antibody in the embodiment of iCeap disclosed in Non-Patent Document 1. Conventionally known anti-EpCAM monoclonal antibodies include VU-1D9 antibody and the like along with these HEA-125 antibody and EBA-1 antibody, and all are established by immunizing mice against tumor cell strains. It has been conventionally known that HEA-125 antibody is specific to human EpCAM, while EBA-1 antibody shows crossreactivity against other animals. Therefore, it has been suggested that an epitope of the human EpCAM protein which HEA-125 antibody recognizes is different from the epitope for EBA-1 antibody. Because of this, these two types of anti-EpCAM monoclonal antibody are each adopted as the first antibody and the second antibody in Non-Patent Document 1.

### SUMMARY

The present inventors intensively carried out examinations to further improve the iCeap disclosed in Non-Patent Document 1. In the process, they surprisingly found the fact that unlike the conventional recognition of those of skill in the art as described above, HEA-125 antibody and EBA-1 antibody recognize epitopes with in a small domain of the human EpCAM protein, and a binding of one antibody impedes a binding of the other. Specifically, they found that these two types of anti-EpCAM monoclonal antibody recognize the N-terminal Epidermal Growth Factor (EGF)-like domain of the human EpCAM protein as an epitope.

The present invention was made based on the above-described unexpected discovery. That is, when iCeap is carried out by using a combination of HEA-125 antibody and EBA-1 antibody, which recognize an identical epitope of the human EpCAM protein, problems may be caused, such as a decline in detection efficiency because these antibodies are competitively counteracted at the epitope site. Hence, an object of the present invention is to provide an anti-EpCAM monoclonal antibody which specifically recognizes an epitope different from the epitope which conventionally known anti-EpCAM monoclonal antibodies recognize (the N-terminal EGF-like domain). Another object of the present invention is to provide a hybridoma which is capable of producing such a monoclonal antibody, and a method for detecting CTC using such a monoclonal antibody and a kit for detecting CTC, which is used for the method.

The present inventors immunized a mouse using HEK293 cells transiently expressing the human EpCAM protein and an immune response against the human EpCAM protein was induced. They produced a hybridoma group by fusing the spleen cells of such mouse and myeloma and searched a hybridoma producing an anti-EpCAM monoclonal antibody which specifically recognizes an epitope different from the N-terminal EGF-like domain of the human EpCAM protein. Consequently, they succeeded in selecting a clone of a hybridoma satisfying this requirement, and succeeded in acquiring a desired monoclonal antibody from such hybridoma, thereby completing the present invention.

That is, according to the first aspect of the present invention, there is provided a monoclonal antibody to human EpCAM, wherein the monoclonal antibody specifically reacts with the CP (Cysteine-Poor) region of human EpCAM.

It is preferred that the monoclonal antibody in the above first aspect does not react with the N-terminal EGF-like domain of human EpCAM. IgM, which has five times larger molecular size than IgG, provides active groups (more than IgG) for fluorophore, biotin, and the like.

According to the second aspect of the present invention, there is also provided a monoclonal antibody to human EpCAM which monoclonal antibody is produced by hybridoma KIJY2 with accession No. NITE BP-1449.

According to the third aspect of the present invention, there is further provided a monoclonal antibody which can bind to the same epitope as for the monoclonal antibody in the above second aspect.

According to the fourth aspect of the present invention, there is provided a method for detecting circulating tumor cells, the method including the steps of:
(a) collecting circulating tumor cells in blood cells from a subject; and
(b) detecting circulating tumor cells contained in the blood sample using any of the above monoclonal antibodies.

In the detection method in the above fourth aspect, it is preferred that the monoclonal antibody be fluorescently-labeled. In the detection method in the above fourth aspect, it is preferred that a second monoclonal antibody, which specifically reacts with the N-terminal EGF-like domain of human EpCAM, be further used in the step (b). It is preferred that the second monoclonal antibody be bound to magnetic beads. In a particularly preferred embodiment in the above fourth aspect, the step (b) is carried out by intact CTC enumeration and analysis procedure (iCeap).

According to the fifth aspect of the present invention, there is further provided a kit for detecting CTC, which is used for the detection method in the above fourth aspect, wherein the kit for detecting CTC comprises a monoclonal antibody in any of the above first to third aspects.

According to the sixth aspect of the present invention, there is provided hybridoma KIJY2 with accession No. NITE BP-1449.

According to the present invention, there is provided an anti-EpCAM monoclonal antibody which specifically recognizes an epitope different from the epitope which conventionally known anti-EpCAM monoclonal antibodies recognize (the N-terminal EGF-like domain). According to the present invention, there are also provided a hybridoma which is capable of producing such a monoclonal antibody, and a method for detecting CTC using such a monoclonal antibody and a kit for detecting CTC, which is used for the method.

The CP region of the human EpCAM protein which an anti-EpCAM monoclonal antibody newly provided by the present invention recognizes as an epitope has unknown characteristics. Therefore, further development of the study on the structure and function of EpCAM protein is expected by utilizing the anti-EpCAM monoclonal antibody in the present invention. Further improvements in detection sensitivity and detection accuracy in the method for detecting CTC as typified by iCeap are also expected by utilizing the anti-EpCAM monoclonal antibody in the present invention.

### BRIEF DESCRIPTION OF THE DRAWINGS

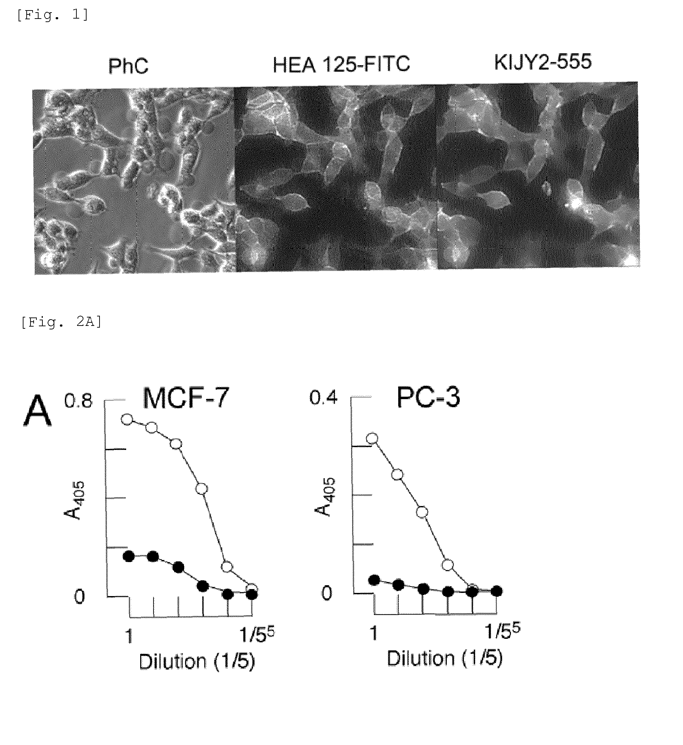

Fig. 1 is a phase-contrast image and fluorescence images of LNCaP cells coimmunostained using HEA-125-FITC and KIJY2-555, which are taken using BIOREVO BZ-9000 (KEYENCE CORPORATION), in the experiment of "immunostaining" in EXAMPLES. In Fig. 1, the left (PhC) shows a phase-contrast image, the middle (HEA 125-FITC) shows a fluorescence image by HEA 125-FITC, and the right (KIJY2-555) shows a fluorescence image by KIJY2-555. Incidentally, 20 X Plan Fl was used as an objective lens. Sites in the cells showing fluorescence in the images shown in Fig. 1 are cell membranes in both HEA-125-FITC and KIJY2-555.
Fig. 2A illustrates the results of the potency test by solid-phase ELISA in the experiment of "ELISA" in EXAMPLES.

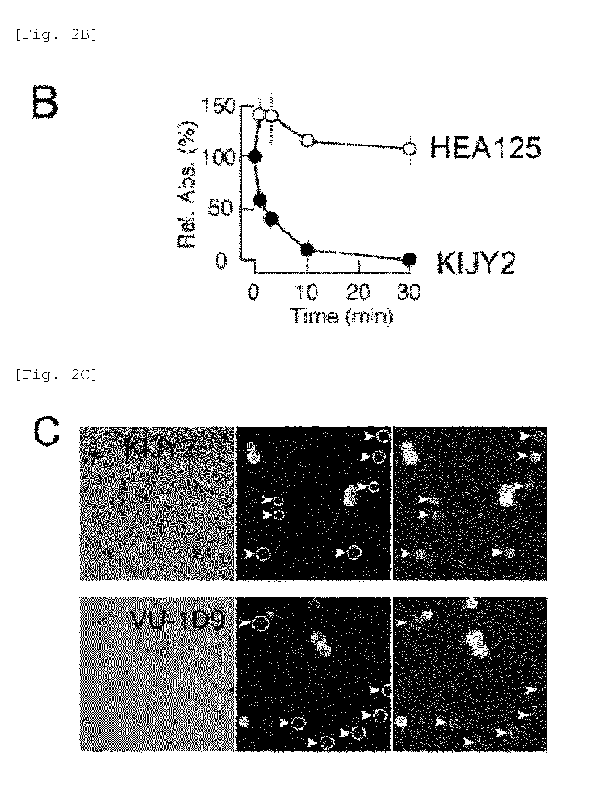

Fig. 2B illustrates the results of the binding specificity test by liquid-phase ELISA in the experiment of "ELISA" in EXAMPLES.
Fig. 2C illustrates the results of visualization of KIJY2 and VU-1D9 antibodies in parallel using a streptavidin-PE conjugate to evaluate the binding ability of KIJY2 antibody to EpCAM expressed on the surface of PC-3 cells in the experiment of "evaluation of the binding ability of KIJY2 antibody to PC-3 cells" in EXAMPLES. In Fig. 2C, the left is the phase-contrast image, the middle is the 12-bit image, and the right is the image after brightness adjustment. The sites of circles shown by arrowheads in the 12-bit images on the middle show the position of EpCAM low-expressing PC-3 cells visualized in the images after brightness adjustment on the left.

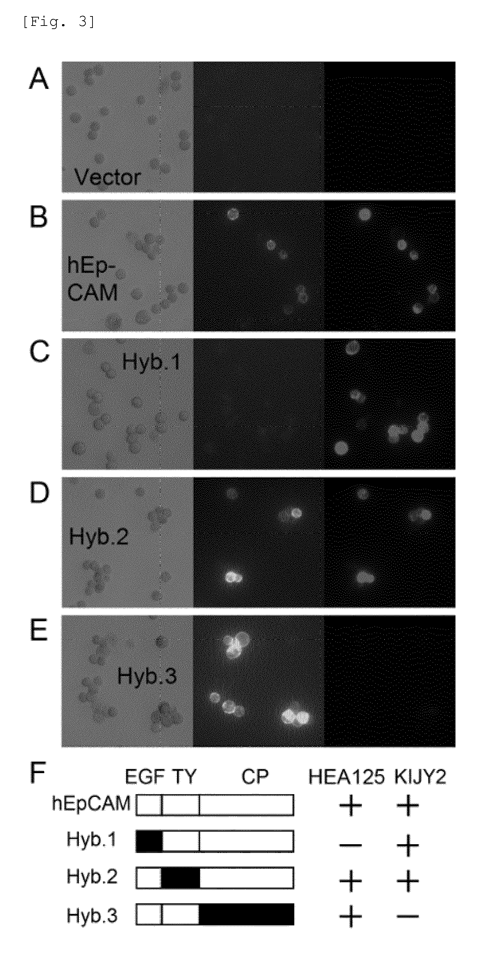

Figs. 3A to 3F are photographs and explanatory diagrams illustrating the results of epitope mapping for the purpose of identifying a binding domain of KIJY2 antibody to human EpCAM in the experiment of "epitope mapping" in EXAMPLES. Each in Figs. 3A to 3F is as follows. A: vector-only, B: full length human EpCAM construct, C to E: EpCAM constructs substituted with the EGF domain, TY domain or CP region of mouse (Hyb.1, Hyb.2 or Hyb.3, respectively). Figs. 3A to 3E show phase-contrast images, FITC fluorescence images and fluorescence images by HiLyte Fluor 555 from the left. Fig. 3F is an explanatory diagram summarizing the contents of the epitope mapping, and the black region shows a region substituted with mouse EpCAM.

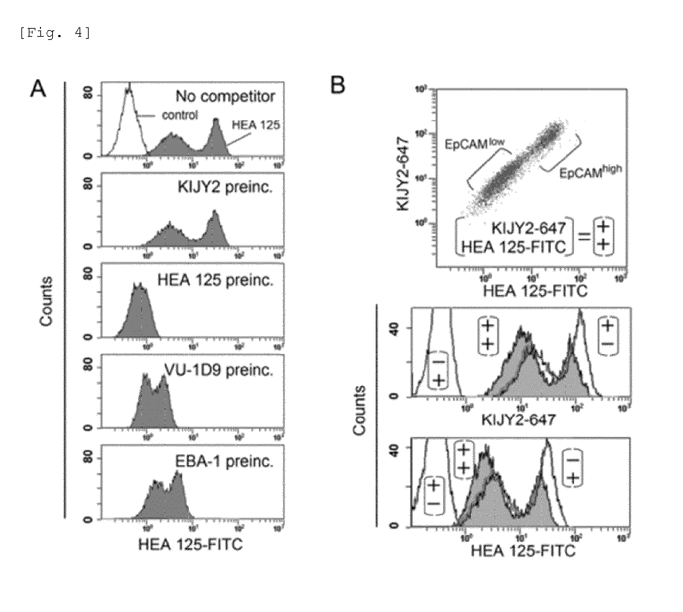

Fig. 4A is diagrams illustrating the results of the competitive binding test on three antibodies, EBA-1 antibody, VU-1D9 antibody and KIJY2 antibody, to PC-3 cells by a flow cytometry (FCM) analysis for the purpose of further determining the reactivity of KIJY2 antibody to PC-3 cells in the experiment of "simultaneous detection of cancer cells" in EXAMPLES. Fig. 4A is histograms showing the results of FCM analysis on treated samples.
Fig. 4B is diagrams illustrating the results of the double staining test of PC-3 cells by HEA 125-FITC or KIJY2 antibody labeled with HiLyte Fluor 647 (KIJY2-647). In Fig. 4B, the upper panel is a scatter gram obtained after fluorescence revision. In Fig. 4B, the lower panels are diagrams showing results when the histogram of the cells stained by both KIJY2-647 and HEA 125-FITC is compared with the histogram of the cells stained by either antigen (the upper panel is comparison with only KIJY2-647 and the lower panel is comparison with HEA 125-FITC).

### DETAILED DESCRIPTION

The embodiments of the present invention will now be described in reference to figures attached.

First, the structure of human EpCAM will be described. Human EpCAM consists of 314 amino acids and is a transmembrane glycoprotein with approximately 40 kDa, and is expressed on the surface of epithelium-derived cells (i.e., many of circulating tumor cells). The extracellular part (EpEx) of this human EpCAM is constituted of the N-terminal EGF-like domain, the TY-like domain and the unidentified cysteine-poor (CP) region adjacent to a single transmembrane helix. This EpEx inhibits E-cadherin and abolishes cadherin-mediated cell-cell adhesion to promote tumor effusion and local invasion. On the other hand, an intracellular short domain of EpCAM (EpICD) released by intramembrane proteolysis plays a role for nuclear signaling together with a transcription factor LEF/TCF, and upregulates c-Myc and cyclin A/E, and promotes cell proliferation.

Conventionally known anti-human EpCAM monoclonal antibodies specifically recognize the N-terminal EGF-like domain because the amino acid sequence of the domain varies between animals, being strongly immunogenic within the EpCAM molecule. Here, "EGF-like domain" will be simply described. An epidermal growth factor (EGF) is a peptide consisting of 53 amino acids, and has a characteristic structure consisting of three disulfide groups formed by six cysteine residues. A large number of proteins have this structure and this structure is referred to as "EGF-like domain".

The gene (cDNA) encoding human EpCAM is already isolated and the amino acid sequence of human EpCAM is also known. SEQ ID NO:1 shows the nucleotide sequence of the CDS (including the stop codon) of the gene encoding human EpCAM (NCBI RefSeq Accession Number: NM_002354) and SEQ ID NO: 2 shows the amino acid sequence of human EpCAM (NCBI RefSeq Accession Number: NP_002345).

atggcgcccc cgcaggtcct cgcgttcggg cttctgcttg ccgcggcgac ggcgactttt 60

gccgcagctc aggaagaatg tgtctgtgaa aactacaagc tggccgtaaa ctgctttgtg 120

aataataatc gtcaatgcca gtgtacttca gttggtgcac aaaatactgt catttgctca 180

aagctggctg ccaaatgttt ggtgatgaag gcagaaatga atggctcaaa acttgggaga 240

agagcaaaac ctgaaggggc cctccagaac aatgatgggc tttatgatcc tgactgcgat 300

gagagcgggc tctttaaggc caagcagtgc aacggcacct ccatgtgctg gtgtgtgaac 360

actgctgggg tcagaagaac agacaaggac actgaaataa cctgctctga gcgagtgaga 420

acctactgga tcatcattga actaaaacac aaagcaagag aaaaacctta tgatagtaaa 480

agtttgcgga ctgcacttca gaaggagatc acaacgcgtt atcaactgga tccaaaattt 540

atcacgagta ttttgtatga gaataatgtt atcactattg atctggttca aaattcttct 600

caaaaaactc agaatgatgt ggacatagct gatgtggctt attattttga aaaagatgtt 660

aaaggtgaat ccttgtttca ttctaagaaa atggacctga cagtaaatgg ggaacaactg 720

gatctggatc ctggtcaaac tttaatttat tatgttgatg aaaaagcacc tgaattctca 780

atgcagggtc taaaagctgg tgttattgct gttattgtgg ttgtggtgat agcagttgtt 840

gctggaattg ttgtgctggt tatttccaga aagaagagaa tggcaaagta tgagaaggct 900

gagataaagg agatgggtga gatgcatagg gaactcaatg cataa 945 (SEQ ID NO:1)

MAPPQVLAFG LLLAAATATF AAAQEECVCE NYKLAVNCFV NNNRQCQCTS VGAQNTVICS 60

KLAAKCLVMK AEMNGSKLGR RAKPEGALQN NDGLYDPDCD ESGLFKAKQC NGTSMCWCVN 120

TAGVRRTDKD TEITCSERVR TYWIIIELKH KAREKPYDSK SLRTALQKEI TTRYQLDPKF 180

ITSILYENNV ITIDLVQNSS QKTQNDVDIA DVAYYFEKDV KGESLFHSKK MDLTVNGEQL 240

DLDPGQTLIY YVDEKAPEFS MQGLKAGVIA VIVVVVIAVV AGIVVLVISR KKRMAKYEKA 300

EIKEMGEMHR ELNA 314 (SEQ ID NO:2)

The monoclonal antibody in the first aspect of the present invention is a monoclonal antibody to human EpCAM, and unlike conventionally known anti-human EpCAM monoclonal antibodies, the monoclonal antibody is characterized by specifically reacting with the CP region of human EpCAM.

It is preferred that the monoclonal antibody in the above first aspect not react with the N-terminal EGF-like domain of human EpCAM. The subclass of the monoclonal antibody in the above first aspect is not particularly limited and is preferably mouse IgM.

As a method for producing the monoclonal antibody in the present aspect, a known method can be directly adopted, and for example, the monoclonal antibody can be produced based on the cell fusion method by Kohler and Milstein. It is generally stated that the spleen cells of an animal such as a mouse in which an immune response to human EpCAM is induced are fused with myeloma to produce a hybridoma group, and a hybridoma producing a desired monoclonal antibody is selected from the hybridoma group. The selected hybridoma is cultured, and a desired monoclonal antibody can be isolated and purified from the culture.

As a method for inducing an immune response to human EpCAM, a general means of protein immunity in which human EpCAM (protein) is inoculated on an animal may be used, and a means of gene immunity in which the human EpCAM gene is administered to an animal may be used.

When an immune response is induced in an animal by protein immunity, a method generally carried out can be adopted without being changed. For example, purified human EpCAM is provided and a mixed solution with an adjuvant is prepared. This mixed solution is subcutaneously injected into e.g., a mouse to induce an immune response to human EpCAM. The mixed solution may be administered several times at intervals as needed for a booster. As described in Example described below, an immune response to human EpCAMmaybe induced by administering cells transiently expressing the human EpCAM protein (e.g., HEK293 cells) to an animal such as a mouse.

Animals to be immunized are not particularly limited and a mouse is preferably used. As a result of this, a monoclonal antibody derived from a mouse can be obtained.

A hybridoma can be produced by the method by Kohler and Milstein. That is, after protein immunization or genetic immunization by the above procedure, a spleen is extracted from an animal in which an immune response to human EpCAM is induced, and spleen cells are collected. The spleen cells and myeloma are fused to produce a hybridoma group. The selection of hybridoma can be carried out, for example, using HAT selective medium. The hybridoma can be cloned, for example, by a limiting dilution method. Thus, the hybridoma producing the anti-human EpCAM monoclonal antibody which reacts with the CP region of human EpCAM (specifically recognizes such region and is bound thereto) may be selected and cloned.

One hybridoma producing the monoclonal antibody in the present invention is named "KIJY2" and has been deposited with International Patent Organism Depositary, National Institute of Technology and Evaluation. The details of deposition are as follows.

Identification reference given by the DEPOSITOR: KIJY2
Accession number:
NITE BP-1449
Date of the deposit (the day of the original deposit):
November 2, 2012

The anti-human EpCAM monoclonal antibody having the above properties can be produced by culturing the selected and cloned hybridoma. A hybridoma may be cultured in the abdominal cavity of an animal or may be cultured in vitro in e.g., a dish. When a hybridoma is cultured in the abdominal cavity of an animal, ascites is collected and a monoclonal antibody can be isolated and purified from the ascites. When a hybridoma is cultured in vitro, a monoclonal antibody can be isolated and purified from the culture fluid.

A monoclonal antibody can be purified by combining known techniques such as various chromatographies, salting-out, dialysis and membrane separation.

A monoclonal antibody which can bind to the same epitope as for the anti-human EpCAM monoclonal antibody produced by KIJY2 (a hybridoma with accession No. NITE BP-1449), i.e., a monoclonal antibody "functionally equal" to the anti-human EpCAMmonoclonal antibody produced by KIJY2, is also encompassed in the present invention. To obtain such monoclonal antibody, for example, the epitope for the anti-human EpCAM monoclonal antibody produced by KIJY2 is analyzed by epitope mapping using e.g., a partial peptide of the human EpCAM protein. Using a synthesized peptide comprising the identified epitope as an antigen, a monoclonal antibody which can bind to the same epitope as for the anti-human EpCAM monoclonal antibody may be obtained. As a method for checking whether epitopes related to two antibodies are the same or not, there is mentioned a method by competition experiment. The anti-human EpCAM monoclonal antibody produced by KIJY2 is for example used as a first antibody. When the binding between the first antibody and human EpCAM is competitively inhibited by a second antibody as a test object, it can be said that the second antibody can bind to the same epitope as for the first antibody.

The monoclonal antibody in the present invention can be used for various uses. The CP region of the human EpCAM protein which the anti-EpCAM monoclonal antibody in the present invention recognizes as an epitope, for example, has unknown characteristics. Therefore, further development of the study on the structure and function of EpCAM protein is expected by utilizing the anti-EpCAM monoclonal antibody in the present invention. Further improvements in detection sensitivity and detection accuracy in the method for detecting circulating tumor cells (CTC) as typified by iCeap are also expected by utilizing the anti-EpCAM monoclonal antibody in the present invention.

A method for detecting circulating tumor cells (CTC) typified by iCeap will be now described.

The method for detecting CTC in the present invention includes the following steps:
(a) collecting a blood sample from a subject; and
(b) detecting CTC contained in the blood sample using any of the above-described anti-EpCAM monoclonal antibodies in the present invention.

In the present invention, a "subject" may be an animal which can be affected by cancer and is preferably human. The detection method in the present invention is particularly preferably carried out for human who is suspected of being affected by cancer or human after being affected by cancer.

"Blood sample" in which circulating tumor cells (CTC) are detected is not particularly limited insofar as, when CTC are contained, such CTC can be detected. It is preferred, however, that a pellet obtained by fusing erythrocytes in a blood sample, followed by centrifugation, and then removing the supernatant be used. Additives such as EDTA potassium salt and heparin may be added to the blood sample for the purpose of preventing coagulation and the like. The timing at which blood is collected from a subject to collect a blood sample is not particularly limited.

As the blood sample used for the detection method in the present invention, a blood sample immediately after being collected from a subject is preferably used for measurement but a preserved one may be also used. The preservation method of a blood sample is not particularly limited on the conditions in which the amount of CTC in a sample is not changed, and preferably under low temperature conditions without freezing, e.g., 0 to 10°C, dark conditions and nonvibrating conditions.

A method for detecting (and determining in some cases) CTC in a blood sample is not particularly limited and a conventionally known means can be adopted as needed. As a preferred embodiment of the present invention, there is mentioned intact CTC enumeration and analysis procedure (iCeap), and in the case of iCeap, the description in e.g., Non-Patent Document 1 can be referred to.

When CTC are detected (and determined) by e.g., iCeap, it is preferred that the above-described anti-EpCAM monoclonal antibody in the present invention be fluorescently-labeled. In this case, fluorochromes for fluorescence labeling are not particularly limited and FITC, HiLyte Fluor 555, HiLyte Fluor 647 and the like can be used.

In the above step (b), detection may be more certainly carried out further using a second monoclonal antibody which specifically reacts with an epitope (e.g., the N-terminal EGF-like domain of EpCAM) different from that for the anti-EpCAM monoclonal antibody in the present invention. In this case, as the second monoclonal antibody, a conventionally known anti-EpCAM monoclonal antibody such as HEA-125, VU-1D9, EBA-1, Ber-EP4, 323/A3 or 311-1K1 can be used. The second monoclonal antibody may be subjected to fluorescent labeling different from that for the anti-EpCAM monoclonal antibody in the present invention or may be bound to magnetic beads. As is the case with iCeap, by using the monoclonal antibody which is bound to magnetic beads as the second monoclonal antibody, the existence of CTC is certainly grasped by two mechanisms of the separation of EpCAM-expressing CTC using magnetism (magnetic separation) and the detection thereof by the anti-EpCAM monoclonal antibody in the present invention (immunofluorescent staining), and the existing amount thereof can be determined in some cases. In some cases, as the anti-EpCAM monoclonal antibody in the present invention, the monoclonal antibody which is bound to magnetic beads is used, and the second monoclonal antibody is fluorescently-labeled by the above-described fluorochrome and may be used in the same manner.

By the detection method in the present invention as described above, there is not the possibility of competitive inhibition to epitopes, for example, by using the anti-EpCAM monoclonal antibody in the present invention and the second monoclonal antibody which recognizes an epitope different from the epitope for the above monoclonal antibody in combination. Therefore, further improvements in the detection efficiency of CTC by e.g., iCeap are expected. The CP region of the human EpCAM protein which the anti-EpCAM monoclonal antibody in the present invention recognizes as an epitope has unknown characteristics. Therefore, further development of the study on the structure and function of EpCAM protein is expected by utilizing the anti-EpCAM monoclonal antibody in the present invention.

According to another aspect of the present invention, there is also provided a kit for detecting CTC, which is used for the above-described method for detecting CTC. This detection kit comprises reagents and the like used for the above-described detection method. Specifically, as reagents contained in such detection kit, there is mentioned the above-described anti-EpCAM monoclonal antibody in the present invention (e.g., the monoclonal antibody which is fluorescently-labeled or that which is bound to magnetic beads). The above-described second monoclonal antibody (e.g. , the monoclonal antibody which is bound to magnetic beads or that which is fluorescently-labeled) can be also contained in such detection kit. The detection kit may further comprise components such as a buffer solution to dilute the above antibodies and collected sample, a cleansing liquid, a secondary antibody, a fluorochrome, a reaction container, positive control, negative control and an instruction book describing a test protocol. These components can be also mixed or combined in advance as needed. By using this detection kit, the CTC detection in the present invention becomes simple, and the kit is very useful for early determination of a medical plan, a prognosis, confirmation of therapeutic effects and the like.

### EXAMPLES

The embodiment of the present invention will be now described in more detail by way of Examples. It should be noted, however, that the scope of the present invention is not limited to the following Examples.

### (Cell strain and culturing)

A mammary gland adenocarcinoma cell strain, MCF-7, was obtained from RIKEN Bioresource Center CELL BANK. Metastatic prostate cancer cell strains, LNCaP and PC-3, were obtained from Cell Resource Center for Biomedical Research, Institute of Development, Aging and Cancer, Tohoku University. These cell strains were cultured in penicillin and streptomycin-added RPMI1640 with 10% FCS (Gibco-Life Technologies). The HEK293 cell strain and transformants thereof were cultured in antibiotic-added D-MEM with 10% FCS (Wako Pure Chemical Industries, Ltd).

### (Expression of EpCAM in HEK293 cell strain)

The cDNA encoding human or mouse EpCAM protein was synthesized based on the nucleotide sequences of NCBI RefSeq Accession Number: NM_002354 (human) and GenBank: BC094465.1 (mouse), respectively, by commissioning Takara Bio Inc. To produce a human-mouse hybrid cDNA, the appropriate site of a restriction enzyme was introduced into a domain without changing the amino acid sequences. The cDNA encoding the human EpCAM protein was cloned into an expression vector pBApo-CMV Neo (Takara Bio Inc). The cDNAs each encoding Hyb.1, Hyb.2 and Hyb.3 polypeptides having the EGF domain, TY domain and CP region of mouse EpCAM, respectively, in the human EpCAM frame were cloned into an expression vector pBApo-CMV Neo. Here, the nucleotide sequences of cDNAs each encoding Hyb.1, Hyb.2 and Hyb.3 are as follows (the underlined portion is the sequence corresponding to mouse EpCAM).

### [cDNA sequence encoding Hyb.1 (having the EGF domain of mouse EpCAM)]

atggcgcccc cgcaggtcct cgcgttcggg cttctgcttg ccgcggcgac ggcgactttt 60

gccgcagctc agagagactg tgtctgtgac aactacaagc tggcaacaag ttgctctctg 120

aatgaatatg gtgaatgcca gtgtacttcc tatggtacac agaatactgt catttgctcc 180

aaactggcgg ccaaatgttt ggtgatgaag gcagaaatga atggctcaaa acttgggaga 240

agagcaaaac ctgaaggggc cctccagaac aatgatgggc tttatgatcc tgactgcgat 300

gagagcgggc tctttaaggc caagcagtgc aacggcacct ccacgtgctg gtgtgtgaac 360

actgctgggg tcagaagaac agacaaggac actgaaataa cctgctctga gcgagtgaga 420

acctactgga tcatcattga actaaaacac aaagcaagag aaaaacctta tgatagtaaa 480

agtttgcgga ctgcacttca gaaggagatc acaacgcgtt atcaactgga tccaaaattt 540

atcacgagta ttttgtatga gaataatgtt atcactattg atctggttca aaattcttct 600

caaaaaactc agaatgatgt ggacatagct gatgtggctt attattttga aaaagatgtt 660

aaaggtgaat ccttgtttca ttctaagaaa atggacctga cagtaaatgg ggaacaactg 720

gatctggatc ctggtcaaac tttaatttat tatgttgatg aaaaagcacc tgaattctca 780

atgcagggtc taaaagctgg tgttattgct gttattgtgg ttgtggtgat agcagttgtt 840

gctggaattg ttgtgctggt tatttccaga aagaagagaa tggcaaagta tgagaaggct 900

gagataaagg agatgggtga gatgcatagg gaactcaatg cataa 945 (SEQ ID NO:3)

### [cDNA sequence encoding Hyb.2 (having the TY domain of mouse EpCAM)]

atggcgcccc cgcaggtcct cgcgttcggg cttctgcttg ccgcggcgac ggcgactttt 60

gccgcagctc aggaagaatg tgtctgtgaa aactacaagc tggccgtaaa ctgctttgtg 120

aataataatc gtcaatgcca gtgtacttca gttggtgcac aaaatactgt catttgctca 180

aagcttgcgt ctaaatgctt ggcgatgaaa gcagaaatga ctcacagcaa gtctgggagg 240

aggataaagc ccgaaggggc gatccagaac aacgatgggc tgtacgaccc cgactgcgac 300

gagcaggggc tcttcaaagc caagcagtgc aacggcaccg ccacgtgctg gtgtgtgaac 360

actgctgggg tcagaagaac agacaaggac actgaaataa cctgctctga gcgagtgaga 420

acctactgga tcatcattga actaaaacac aaagcaagag aaaaacctta tgatagtaaa 480

agtttgcgga ctgcacttca gaaggagatc acaacgcgtt atcaactgga cccaaaattt 540

atcacgagta ttttgtatga gaataatgtt atcactattg atctggttca aaattcttct 600

caaaaaactc agaatgatgt ggacatagct gatgtggctt attattttga aaaagatgtt 660

aaaggtgaat ccttgtttca ttctaagaaa atggacctga cagtaaatgg ggaacaactg 720

gatctggacc ctggtcaaac tttaatttat tatgttgatg aaaaagcacc tgaattctca 780

atgcagggtc taaaagctgg tgttattgct gttattgtgg ttgtggtgat agcagttgtt 840

gctggaattg ttgtgctggt tatttccaga aagaagagaa tggcaaagta tgagaaggct 900

gagataaagg agatgggtga gatgcatagg gaactcaatg cataa 945 (SEQ ID NO:4)

### [cDNA sequence encoding Hyb.3 (having the CP region of mouse EpCAM (longer by a codon (3 bases) than that of human))]

atggcgcccc cgcaggtcct cgcgttcggg cttctgcttg ccgcggcgac ggcgactttt 60

gccgcagctc aggaagaatg tgtctgtgaa aactacaagc tggccgtaaa ctgctttgtg 120

aataataatc gtcaatgcca gtgtacttca gttggtgcac aaaatactgt catttgctca 180

aagctggctg ccaaatgttt ggtgatgaag gcagaaatga atggctcaaa acttgggaga 240

agagcaaaac ctgaaggggc cctccagaac aatgatgggc tttatgatcc tgactgcgat 300

gagagcgggc tctttaaggc caagcagtgc aacggcacct ccacgtgctg gtgtgtcaac 360

accgccggag tccgaagaac cgacaaggac acggagatca cgtgctccga gcgcgtgagg 420

acctactgga tcatcattga actaaaacac aaagaaagag aaagccccta cgaccatcag 480

agcttgcaga ctgcgcttca agaggcgttc acatctcgat ataagctgaa tcagaaattt 540

atcaaaaaca ttatgtatga gaataatgtt atcaccattg atctgatgca aaactcttct 600

cagaaaacac aagacgacgt ggacatagct gatgtggctt actattttga aaaagatgtg 660

aagggggagt ccctgttcca ttcttctaag agcatggacc tgagagtgaa cggagagccg 720

ctcgatctgg accccgggca gactctgatt tactacgttg atgaaaaggc acccgaattc 780

tcaatgcagg gtctaaaagc tggtgttatt gctgttattg tggttgtggt gatagcagtt 840

gttgctggaa ttgttgtgct ggttatttcc agaaagaaga gaatggcaaa gtatgagaag 900

gctgagataa aggagatggg tgagatgcat agggaactca atgcataa 948 (SEQ ID NO:5)

Thereafter, each expression vector was transfected into HEK293 cells using X-tremeGENE HP (Roche) as a transfection reagent. Each stable HEK293-EpCAM clone was established by selection using G418 (Roche).

### (Immunization of mouse and production of a hybridoma clone)

A Balb/c mouse was immunized with transient EpCAM-expressing HEK293 cells establishedabove. Theproductionof a hybridoma clone and purification of a monoclonal antibody were carried out as commissioned research in Nippon Biotest Laboratories Inc. To generally state this means, spleen was first extracted from the immunized mouse, spleen cells were collected and these were fused with a myeloma cell strain P3U1 to produce a hybridoma group. From the hybridoma group thus obtained, a desired hybridoma clone was screened using a cell strain overexpressing EpCAM, MCF-7 cell strain. Positive samples were further screened using HEK293 negative parent cells and HEK293 positive cells stably expressing EpCAM as standards. The resulting hybridoma clone was named "KIJY2" and cultured in a serum-free culture medium. An antibody (KIJY2 antibody) produced by this was homogenously purified. The KIJY2 antibody belongs to IgM which does not have significant non-specific binding.

### (Immunostaining)

The purified KIJY2 antibody was fluorescently-labeled using an N-hydroxy succinimide (NHS) labeling kit (DOJINDO LABORATORIES) according to the maker's manual. LNCaP cells were cultured in a 35-mm culture dish, washed using PBS and allowed to react with fluorochrome-binding KIJY2 antibody obtained above. An appropriate dilution of HEA-125-FITC (Miltenyi Biotec) and concentration of HiLyte Fluor 555-binding KIJY2 antibody (KIJY2-555) were determined 1/25 and 0.4 µg/mL, respectively, relative to 1 to 2 X 10⁵ cultured cells/sample.

A phase-contrast image and fluorescence images of LNCaP cells coimmunostained using HEA-125-FITC and KIJY2-555 were obtained by BIOREVO BZ-9000 (KEYENCE CORPORATION).

The photographs showing the obtained images are shown in Fig. 1. In Fig. 1, the left (PhC) shows a phase-contrast image, the middle (HEA 125-FITC) shows a fluorescence image by HEA 125-FITC, and the right (KIJY2-555) shows a fluorescence image by KIJY2-555. Incidentally, 20 X Plan Fl was used as an objective lens. Sites in the cells showing fluorescence in the images shown in Fig. 1 are cell membranes in both HEA-125-FITC and KIJY2-555.

### (ELISA)

MCF-7 cells and PC-3 cells (up to 5 X 10⁴/mL) expressing EpCAM were each seeded on a 96-well poly-L-lysine coated microplate (SUMITOMO BAKELITE CO., LTD.) and cultured for 24 hours. Adherent cells were immobilized using 4% paraformaldehyde (PFA) in PBS solution for 15 minutes, and by using this as an antigen in ELISA, a hybridoma clone was selected, a potency test was carried out and a titration curve was created.

Solid-phase ELISA was carried out using HRP Protein Detector Microwell Kit (KPL). In this case, HEA-125 (IgG1, ab46714, Abcam, UK), an anti-EpCAM monoclonal antibody, was used as positive control. Potency against MCF-7 cells and PC-3 cells described in documents was determined by beginning with undiluted HEA-125, or 50 µg/mL KIJY1 antibody or KIJY2 antibody. The secondary antibody was substituted with an anti-mouse IgM-HRP equivalent (Sigma-Aldrich) for the detection of KIJY2 antibody.

The results of the potency test by this solid-phase ELISA are shown in Fig. 2A. As shown in Fig. 2A, it is revealed that when HEA-125 is used, the antibody with a higher concentration is required for detecting PC-3 cells than for MCF-7 cells. Unexpectedly, KIJY2 antibody was bound to MCF-7 cells in some degree, but was hardly bound to PC-3 cells. This showed that the epitope for KIJY2 antibody was very highly susceptible to PFA treatment, while the binding ability thereof to untouched (unimmobilized) cells could be maintained.

Next, liquid-phase ELISA was carried out by the following means for the purpose of testing the binding specificity of unimmobilized cells in a suspension: PC-3 cells exfoliated using TrypLE Express (Life Technologies) were suspended in PBS with 4% PFA, and the obtained suspension was distributed to a low-binding microtube (PROTEOSAVE SS, SUMITOMO BAKELITE CO., LTD.) at 10⁵ cells/sample. Next, this was quickly diluted in a 10 X volume medium, followed by washing with activator-free PBS twice, and the obtained cell pellet was used for liquid-phase ELISA. For liquid-phase ELISA, M-Buffer (PBS with 0.5% BSA and 2 mM EDTA) was used. To test paraformaldehyde sensitivity, the excessive amount of HEA-125 (1/10 dilution) or KIJY2 antibody (5 µg/mL) was maintained under analysis conditions, and the same experiment was carried out three times.

The results of binding specificity test by liquid-phase ELISA are shown in Fig. 2B. As shown in Fig. 2B, the epitope for HEA-125 was slightly exposed immediately after the treatment by 4% PFA (up to 1.5-fold). The epitope for KIJY2 antibody meanwhile quickly disappeared after the treatment by 4% PFA. It is known that EpCAM can form a multimer and can interact with other proteins. These properties of EpCAM can inhibit the possibility of KIJY2 antibody approaching to an epitope immobilized by PFA. As another possibility, it is also thought that the structure peculiar to EpCAM is broken down by PFA treatment. Anyway, it should be noted that cell immobilization can affect immunoreactivity.

### (Evaluation of binding ability of KIJY2 antibody to PC-3 cells)

To evaluate binding ability of KIJY2 antibody to EpCAM expressed on the surface of natural cancer cells (PC-3 cells), KIJY2 and VU-1D9 antibodies were visualized in parallel. Specifically, cells bound to each antibody were visualized using a streptavidin-PE conjugate. The EpCAM level of PC-3 cells is relatively low among known EpCAM positive cancer cell strains, and PC-3 cells include subpopulations with different expression levels of EpCAM. Because of this, in this experiment, PC-3 cells were selected as a cancer cell strain which is difficult to detect with consideration to practical application of CTC detection.

Specifically, VU-1D9 (Santa Cruz Biotechnology, Inc.), a BSA-free anti-EpCAM monoclonal antibody, and KIJY2 antibody (each 100 µg) were biotinylated in parallel using a biotin labeling kit-NH2 (DOJINDO LABORATORIES). PC-3 cells were incubated in the presence of abiotinylatedmonoclonal antibody (0.4µg/10⁵cells) inM-Buffer at room temperature for 30 minutes. The cells were washed with PBS and then mixed with streptavidin-PE (Vector Laboratories, Inc.) at a recommended dilution (1/100) for 15 minutes. The cells in the suspension were left to stand in a disposable hemacytometer C-Chip (Seoul, South Korea) and analyzed by BZ-9000 (Objective lens: 10 X Plan Apo).

HEA 125-FITC (4 µL) and KIJY2 (0.4 µg) were mixed with a HEK293 transient transformant (1.5 to 2 X 10⁵ cells/sample) in a 100 µL cell suspension and this was monitored in C-Chip as described above.

PC-3 cells were visualized using a streptavidin-PE conjugate. The results are shown in Fig. 2C. In Fig. 2C, the upper row shows the results using KIJY2 antibody and the lower row shows the results using UV-1D9. In Fig. 2C, the left is the phase-contrast image, the middle is the 12-bit image, and the right is the image after brightness adjustment. The sites of circles shown by arrowheads in the 12-bit images on the middle show the position of EpCAM low-expressing PC-3 cells visualized in the images after brightness adjustment on the left. The results shown in Fig. 2C reveal that both monoclonal antibodies, KIJY2 antibody and UV-1D9, can detect subpopulations of PC-3 cells in which the expression level of EpCAM is high (middle) and PC-3 cells in which the expression level is low (right) in the same manner. This showed that KIJY2 antibody could detect intact cancer cells as effectively as VU-1D9 antibody regardless of the expression level of EpCAM in cells.

### (Epitope mapping)

Next, epitope mapping was carried out for the purpose of identifying the binding domain of KIJY2 antibody to human EpCAM.

Here, a human-mouse EpCAM hybrid cDNA construct was prepared to obtain a hybrid protein in which the three-dimensional molecule structure of EpCAM protein was maintained without being changed. Specifically, the EGF domain, the TY domain or the CP region of the human EpCAM protein was substituted with each counterpart of mouse to obtain Hyb.1, Hyb.2 and Hyb.3. HEK293 cells temporarily expressing these chimera proteins were simultaneously stained using HEA125-FITC and KIJY2-555.

The results of staining are shown in Figs. 3A to 3F. Each in Figs. 3A to 3F is as follows. A: vector-only, B: full length human EpCAM construct, and C to E: EpCAM constructs substituted with the EGF domain, TY domain or CP region of mouse (Hyb.1, Hyb.2 or Hyb.3, respectively). Figs. 3A to 3E show phase-contrast images, FITC fluorescence images and fluorescence images by HiLyte Fluor 555 from the left. Incidentally, 20 X Plan Apo was used as an objective lens. Fig. 3F is an explanatory diagram summarizing the contents of epitope mapping, and the black region shows a region substituted with mouse EpCAM.

The results shown in Figs. 3A to 3F show that HEA-125 and KIJY2 antibodies each recognize the different portions of EpCAM molecule. Specifically, as shown in Fig. 3B, complete EpCAM was stained by both HEA 125-FITC and KIJY2-555. As shown in Fig. 3C, Hyb.1, which replaces human EGF domain with mouse counterpart, or lacks human sequence of EGF domain, was stained by KIJY2-555 but was not stained by HEA-125. The results support conventional knowledge that HEA-125 recognizes the EGF domain of human EpCAM. As shown in Fig. 3E, Hyb.3, which replaces human CP region with mouse counterpart, or lacks human sequence of CP region, was stained by HEA-125 but was not stained by KIJY2-555. The results showed that KIJY2 antibody specifically recognized the CP region of human EpCAM and reacted with the region.

A phenomenon is known, in which EpCAM is cut from a cell membrane. To check this cutting, KIJY2 antibody, which is a monoclonal antibody specific to the CP region adjacent to a membrane, is expected to be useful for evaluating EpCAM cutting. In addition, 311-1K1, which is an anti-EpCAM monoclonal antibody specific to the CP region, does not stain undenatured EpCAM and does not act well in FCM, but has been used in early reports. KIJY2 antibody acts well in FCM and thus is bound to undenatured EpCAM better. The images shown in Fig. 2C show that KIJY2 antibody can detect EpCAM low-expressing PC-3 cells (EpCAM^{low}) as is the case with VU-1D9 antibody. From these images, it is also realized that KIJY2 and VU-1D9 antibodies are colocalized.

### (Simultaneous detection of cancer cells)

FCM analysis was carried out to further determine the reactivity of KIJY2 antibody to PC-3 cells. First, competitive binding of three types of antibody, EBA-1, an anti-human EpCAM monoclonal antibody in which an epitope thereof has not been conventionally mapped, VU-1D9, an anti-human EpCAM monoclonal antibody specific to the EGF domain, and KIJY2 antibody, which is realized to be specific to the CP region as described above, to PC-3 cells was tested.

Specifically, first for 60 minutes, PC-3 cells (2 X 10⁵ cells in a 100 µL sample) were preincubated in M-Buffer together with 100 µL of non-diluted HEA-125 (Abcam, ab46714), 4 µg of VU-1D9, 4 µg of EBA-1 (Santa Cruz Biotechnology, Inc.) or 4 µg of KIJY2 antibody (all are excessive amounts), or preincubated with the buffer alone. After that, 10 µL of HEA 125-FITC was mixed therewith for 15 minutes.

The results (histogram) of FCM analysis of samples treated as above are shown in Fig. 4A. As shown in Fig. 4A, it was repeatedly observed that only PC-3 cells stained by HEA 125-FITC showed a histogram having a double peak (Fig. 4A, the top panel). Incidentally, any specific subpopulation was not distinguished by FS/SS scatter gram (not shown). It is believed that because the same histogram has conventionally reported, the expression of EpCAM of a different degree is the original properties of PC-3 cell strain. When the cells were preincubated together with HEA-125, VU-1D9 or EBA-1 antibody, a positive signal disappeared or was much reduced (Fig. 4A, lower three panels). These results showed that EBA-1 was a monoclonal antibody specific to the EGF domain of EpCAM and EBA-1 and VU-1D9 antibodies were competitively bound to the epitope for HEA-125 at the EGF domain. On the other hand, FITC histogram of a sample preincubated with KIJY2 antibody was hardly changed (Fig. 4A, the second panel from the top). From this, it is revealed that the prebinding of KIJY2 antibody did not disturb the subsequent binding reaction of HEA-125. That is, the epitope for KIJY2 antibody was confirmed to be different from that for HEA-125.

Next, the double staining test of PC-3 cells was carried out by HEA 125-FITC or KIJY2 antibody labeled with HiLyte Fluor 647 (KIJY2-647).

Specifically, PC-3 cells were simultaneously mixed with HEA125-FITC (10 µL) and KIJY2-647 (0.4 µg) for 30 minutes. After washing, the cells were analyzed by Cytomics FC 500 Flow Cytometry System (Beckman Coulter, Inc). The excitation wavelengths were 488 nm for FITC and 633 nm for HiLyte Fluor 647. The detection channel was FL1 (495 to 555 nm) and FL4 (645 to 705 nm). For comparison, data with every 10000 cases were acquired.

The results are shown in Fig. 4B. In Fig. 4B, the upper panel is a scatter gram obtained after fluorescence revision. As shown in this scatter gram, both the EpCAM high-expressing subpopulation (EpCAM^{high}) and the EpCAM low-expressing subpopulation (EpCAM^{low}) in the cell population of PC-3 cell strain could be co-stained.

In Fig. 4B, the lower panels are diagrams showing results when the histogram of the cells stained by both KIJY2-647 and HEA 125-FITC is compared with the histogram of the cells stained by either antigen (the upper panel is comparison with only KIJY2-647 and the lower panel is comparison with HEA 125-FITC). As shown in these histograms, a slight decrease in fluorescent strength and a distribution shift from EpCAM^{high} to EpCAM^{low} in both FITC and HiLyte Fluor 647 were observed on the histogram stained using each single dye. It is known that EpCAM tends to form a dimer or a tetramer, and KIJY2 antibody does not recognize EpCAM immobilized with PFA as described above (Fig. 2B). Thus, KIJY2 antibody can more preferentially recognize EpCAM molecules in the free state on a membrane than molecules in which a structure by another antibody or PFA cross-linking is immobilized.

It should be noted that KIJY2-647 can distinguish all PC-3 cells from background signals regardless of the existence of HEA-125. According to conventional reports in which PC-3 expresses 51,667 EpCAM/cell, the lower limit of EpCAM low-expressing cells is presumed to be approximately 2, 000 EpCAM/cell by FCM analysis. This value is enough to cover the expression amount of EpCAM (9, 900 to 246,000 molecules/cell) in EpCAM positive CTC presumed from metastatic cancer patients.

### [Explanation of Sequence Listing]

### [SEQ ID NO:1]

SEQ ID NO: 1 is the nucleotide sequence of the CDS (comprising the stop codon) of the gene encoding human EpCAM (NCBI RefSeq Accession Number: NM_002354).

### [SEQ ID NO:2]

SEQ ID NO:2 is the amino acid sequence of human EpCAM (NCBI RefSeq Accession Number: NP_002345).

### [SEQ ID NO:3]

SEQ ID NO:3 is the nucleotide sequence of cDNA encoding Hyb. 1 (having the EGF domain of mouse EpCAM) synthesized in Example.

### [SEQ ID NO:4]

SEQ ID NO:4 is the nucleotide sequence of cDNA encoding Hyb.2 (having the TY domain of mouse EpCAM) synthesized in Example.

### [SEQ ID NO:5]

SEQ ID NO:5 is the nucleotide sequence of cDNA encoding Hyb.3 (having the CP region of mouse EpCAM) synthesized in Example.

## Claims

1. A monoclonal antibody to the human epithelial cell adhesion molecule (EpCAM), wherein the monoclonal antibody specifically reacts with the CP region of the human epithelial cell adhesion molecule (EpCAM).

2. The monoclonal antibody according to claim 1, wherein the monoclonal antibody does not react with the N-terminal EGF-like domain of the human epithelial cell adhesion molecule (EpCAM).

3. The monoclonal antibody according to claim 1 or 2, wherein the subclass is mouse IgM.

4. A monoclonal antibody to the human epithelial cell adhesion molecule (EpCAM), wherein the monoclonal antibody is produced by hybridoma KIJY2 with accession No. NITE BP-1449.

5. A monoclonal antibody which can bind to the same epitope as for the monoclonal antibody according to claim 4.

6. A detection method for circulating tumor cells, the method comprising the steps of:
(a) collecting circulating tumor cells in blood cells from a subject; and
(b) detecting circulating tumor cells contained in the blood sample using a monoclonal antibody according to any one of claims 1 to 5.

7. The detection method according to claim 6, wherein the monoclonal antibody is fluorescently-labeled.

8. The detection method according to claim 6 or 7, which further uses a second monoclonal antibody which specifically reacts with the N-terminal EGF-like domain of the human epithelial cell adhesion molecule (EpCAM) in the step (b).

9. The detection method according to claim 8, wherein the second monoclonal antibody is bound to magnetic beads.

10. The detection method according to any one of claims 6 to 9, wherein the step (b) is carried out by intact CTC enumeration and analysis procedure (iCeap).

11. A kit for detecting circulating tumor cells, which is used for a detection method according to any one of claims 6 to 10, wherein the kit for detecting circulating tumor cells comprises a monoclonal antibody according to any one of claims 1 to 5.

12. Hybridoma KIJY2 with accession No. NITE BP-1449.
